# EUROPEAN PATENT SPECIFICATION

(11) **EP 1 929 951 B1**
(45) Date of publication and mention of the grant of the patent: **29.07.2009**
(21) Application number: 06291720.8
(22) Date of filing: 31.10.2006
(51) Int. Cl.: A61B 7/00, A61B 5/103, G01N 19/02, G01N 29/14, G01N 29/22, G01N 29/44

(54) **Acoustic system and method for evaluation skin texture**
System und Verfahren zur akustischen Bestimmung von Hauttexturen
Système et procédé pour la détermination acoustique des textures de la peau

(43) Date of publication of application: 11.06.2008
(73) Proprietor: Johnson & Johnson Consumer France SAS, 92787 Issy les Moulineaux Cedex 9 (FR)
(72) Inventor: Bruère, Valérie, 95210 Saint Gratien (FR); Issachar, Nathalie, 75016 Paris (FR); Lindermeier, Tanja, 78230 Le Pecq (FR); Nkengne, Alex, 92240 Malakoff (FR)
(74) Representative: Metten, Karl-Heinz

(56) References cited:
- WO-A-20/05085805
- DE-A1- 4 330 752
- FR-A1- 2 811 764

## Description

The present invention relates to an acoustic system and method for evaluating skin texture. The system and method may be used, for example, for determining skin type, diagnosing appropriate skin treatments and assessing skin changes. The method is particularly useful for demonstrating the effectiveness of cosmetic products for improving skin surface properties, such as smoothness and wrinkle and fine line reduction.

### Background of the Invention

Determination of skin surface texture is of great importance in the field of dermatology. Skin texture can be used for skin diagnostics and evaluation of therapeutic or cosmetic treatments. Evaluation of skin texture by means of visual inspection or touch has been performed for centuries. Experienced professionals such as dermatologists can assess changes in the skin with great accuracy. With advances in technology, the ability to evaluate skin texture has become a powerful adjunct methodology when evaluating a skin condition. Some technologies can provide quantitative measurements, improving the accuracy of the diagnostic tool.

WO 2005/046474 discloses an acoustic emission system for objectively measuring tactile skin attributes and methods of using the same. The system includes a means for generating an acoustic emission signal from skin; a means for collecting, storing and displaying said emission signal; and a means for correlating said emission signal with an attribute of said skin. In particular, the system uses acoustic signals generated from the frictional forces generated from skin/skin contact (rubbing of the hand or finger on another skin part). These generate vibration patterns sensed by a probe placed near the skin/skin contact area.

However, this method introduces several variables that affect the measurements. First, as acknowledged in the document," since acoustic emission depends on the speed of the rubbing and the pressure at the skin/skin contact, several recordings are performed for a given subject..." Moreover, the method is performed in an open area (either in air or water) and therefore does not filter out background noise effectively, especially when performed in air.

In FR 2 811 764 A1 an apparatus for evaluating the acoustic properties of friction is described. This apparatus comprises a housing with an opening through which the top fraction of a flexible brush can be passed from the inside of the housing to the outside in order to contact the surface to be analyzed. Inside the housing the brush is attached to a holding arm which in turn is connected to a sensing head. With the apparatus of FR 2 811 764 A1 the surface of the specimen to be subjected to testing is lying outside the housing.

DE 43 30 742 A1 describes an apparatus for testing the acoustic properties of a specimen. Sound is generated on the surface of said specimen. The sound generated at said specimen is detected with an acoustic sensor while the specimen is subjected to sound. The acoustic sensor is part of the apparatus to which also belongs the device for generating sound.

WO 2005/085805 A1 is about a sensor for the quantitative measurement of the feel of a surface which comprises a prehensile envelope, a hollow contact body for bringing into contact with the surface on a sensing zone, first acoustic detection elements to detect noises emitted by the hollow body on contact with the sensing zone, and second mechanical detection elements embodied for measurement of the normal pressure or the normal pressure on the rubbing force exerted by the surface on the hollow body. By use of such a sensor the triboacoustic properties of the skin or phanera, of textiles, leather, plastic materials or any other material shall be detectable.

Accordingly, an improved acoustic system and method for evaluating skin texture have been developed that minimize the interference of external sounds and provide excellent sensitivity to sound. The system comprises an acoustic isolation box to remove background noise from the evaluation area. This is important in that the sounds being measured have a very low volume. In addition, the system comprises a guiding device attached to a sound generator for generating a uniform and consistent measurement.

### Summary of the Invention

The invention provides an acoustic system for evaluating skin texture according to claim 1.

The invention also provides a method for evaluating skin texture according to claim 9.

### Brief Description of the Drawings

FIG. 1 is a side view of a system according to the present invention.
FIG. 2 is a front view of a system according to the present invention.

### Detailed Description of the Invention

The acoustic isolation box is sized appropriately to receive a portion of skin. For example, the acoustic isolation box may be sized to accommodate a forearm. It comprises an opening for inserting the skin. The acoustic isolation box is made to minimize sounds external to the box, thereby maximizing the sensitivity of the system to the sound generated inside the box from the skin.

The acoustic isolation box may be made from materials known in the art. For example, the box may be made with polyurethane foam (for instance 35 mm) on the inside, a heavy bituminous membrane (for instance 5.5 mm) and a cellulose fiber membrane (for instance 38 mm), such as Isofon on the outside.

In one embodiment, the acoustic isolation box is capable of reducing external noise by at least 5 dB inside the box.

The guiding device moves the sound generator, discussed below, along the skin being evaluated in a sliding fashion. The guiding device may, for example, be a linear translation table. In one embodiment, the guiding device moves the sound generator longitudinally along the skin. The guiding device can be located within the isolation box or at least partially outside said box. In the latter case it is for example the element which provides for the attachment of the sound generator
to the guiding device which is inside the isolation box for example by way of a sealed or sealable slit or hole.

The system may optionally comprise a motor for moving the guiding device. One suitable example of a combination motor and guiding device is the Mac23 brushless motor from Midi Ingenierie. The motor may have a controller card, such as the Mac24-1 having 10000 points resolution from Midi Ingenierie. The motor may be controlled by Macsim V 5 software from Midi Ingenierie.

The sound generator may be any type of object that generates sound when dragged across or slidably contacted with the skin. For example, the sound generator may be in the form of a card made of paper, cardboard, metal, plastic, or any other suitable material that does not harm the skin. The shape and thickness of the sound generator may be adjusted depending on the skin surface to be tested and the flexibility of the material from which it is made. Generally, in the case of a card, the card may be from 100 micrometers to 0.5 centimeters thick.

The sound generator is attached to the guiding device by methods available in the art. For example, the sound generator may be attached to the guiding device by springs. The sound generator is placed entirely within the isolation box.

The sound generator is preferably designed to deliver a constant pressure against the skin of from 0.001 Pa to 10 Pa.

Any means for capturing sound may be utilized in the system of the present invention. The means for capturing sound is usually placed at least partially within the isolation box. For example, a Gras-40AE 0.5 inch microphone from Oros France is suitable. The microphone may have a preamplifier, such as a Gras-26CA 0.5 inch preamplifier from Oros France.

The means for capturing sound should be sensitive to a signal with a frequency from 0.5 Hz to 22000 Hz.

In one embodiment, the means for capturing sound is connected to a computer. For example, the means for capturing sound may be a microphone that utilizes a sound card, for example a Duo USB 24 bits/96 kHz sound card from M-Audio that is useful for connection to a computer. Sound recorder software, such as Praat Version 4.2.19 may be useful for capturing sounds and evaluating them on the computer.

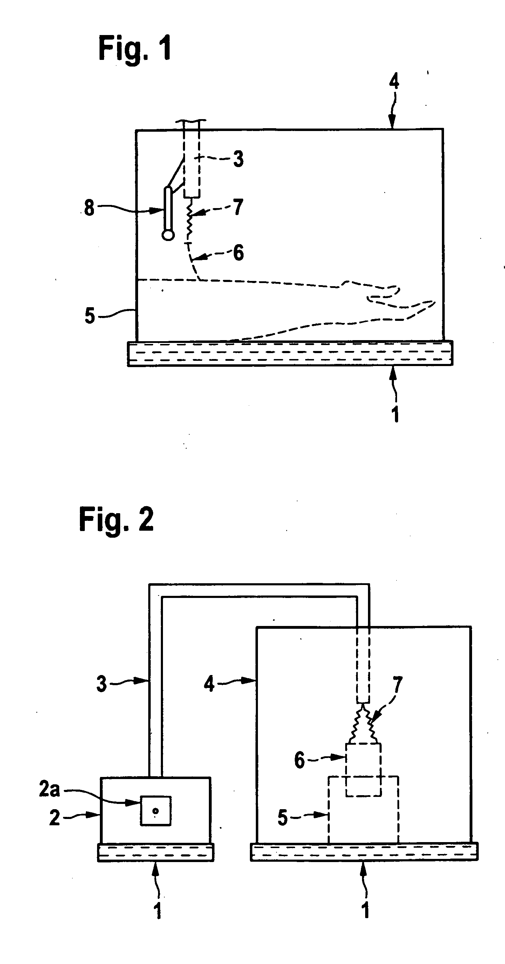

The system according to the present invention may be better understood with reference to the Figures. In the embodiment seen in FIG. 1 and FIG. 2, the system 10 includes a motor 2a and guiding device 2. The system may include a support 1, e.g. a wooden support, for resting the portion of skin to be tested. In this embodiment, a metal rod 3 is connected to the motor 2a and guiding device 2. At the distal end of the rod 3 are attachment means 7 for sound generator 6. In the example illustrated, attachment means 7 are springs and the sound generator 6 is a card. Means for capturing sound 8, e.g. a microphone, can be attached to rod 3. Acoustic isolation box 4 surrounds the testing area, including the means for capturing sound and sound generator. The acoustic isolation box is equipped with opening 5 for insertion of the skin to be measured, e.g. the skin of a forearm.

According to the invention, the texture of a sample of skin may be evaluated using this system as follows. The sample is placed into contact with the sound generator inside the acoustic isolation box; the guiding device is moved such that the sound generator slidably contacts the skin to generate sound; the sound is captured by the means for capturing sound; and the captured sound compared to a predetermined standard.

The captured sound may be compared to the predetermined standard, for example by volume in decibels ("dB"), or by processing the signal through an algorithm. Suitable methods of processing sound signals are known and include Fourier transformation, wavelet, and discrete cosine transformation. Data mining techniques, such as neural networks, support vector machines, partial least square regression, and principle component analysis may be utilized to find the best sound descriptors.

Using the system and method of the present invention, for example, the sound of a baby's skin may be recorded as a predetermined standard. An adult's skin sound may then be captured and compared to the baby's skin sound according to the invention.

In one embodiment, the system and method enable a consumer to determine if one or more skin care products are improving her skin texture. For example, a portion of her skin may evaluated according to the invention before the use of a certain skin care product, for example a moisturizer, and then be re-evaluated after use of the skin care product. A baby's skin sound may be used as the predetermined standard. If the consumer's skin sound has become more like the baby's skin sound, it may be concluded that the consumer's skin texture has improved.

The following example further illustrates the claimed invention.

### Example 1

An acoustic system according to the invention was assembled as follows. A Mac23 brushless motor from Midi Ingenierie having a Mac24-1 controller card (also from Midi Ingenierie) was used. The controller card had 10000 points resolution. The motor was controlled by Macsim V 5 software from Midi Ingenierie. A cardboard care was used as a sound generator. The sound generator was attached to the motor by springs. The motor and card were designed to deliver a constant pressure against the skin of from 0.001 Pa to 10 Pa.

An acoustic isolation box was sized appropriately to receive a human forearm and hold the components described above. The acoustic isolation box was made from 35 mm of polyurethane foam on the inside, 5.5 mm of a heavy bituminous membrane and 38 mm of Isofon cellulose fiber membrane on the outside. The acoustic isolation box reduced external noise by greater than 5 dB. A Gras-40AE 0.5 inch microphone from Oros France was used to capture the sound. The microphone had a Gras-26CA 0.5 preamplifier. The microphone was sensitive to a signal with a frequency from 0.5 Hz to 22000 Hz. The microphone utilized a Duo USB 24 bits/96 kHz sound card from M-Audio, and was connected to a computer. Praat Version 4.2.19 sound recorder software was used for capturing sounds and comparing them on the computer.

Experiments were performed to determine skin texture differences between old and young skin using this system. Adult human subjects with normal skin, dry skin, and moisturized skin were tested. Frequency in Hz was determined as a function of Power Spectral Density in dB/Hz. The data for moisturized skin was clearly below the data for dry skin and normal skin.

In a separate experiment, baby and adult skin were compared for sound intensities in dB. The baby skin generated 7.48 dB of sound while the adult skin generated 10.33 dB of sound. The adult skin was then treated with a moisturizing lotion. After treatment, the adult skin generated 8.17 dB of sound.

## Claims

1. An acoustic system (10) for evaluating skin texture, comprising an acoustic isolation box (4), a guiding device (2) having a sound generator (6) attached thereto, the sound generator being adapted to generate sound when dragged across or slidably contacted with the skin, and means (8) for capturing sound, and wherein the isolation box is equipped with at least one opening (5) for insertion of the skin to be measured, and wherein the sound generator is placed within the isolation box.

2. The system according to claim 1 further comprising a motor (20) attached to the guiding device.

3. The system according to claim 1 or 2 wherein the sound generator is a card.

4. The system according to claim 3 wherein the card is attached to the guiding device by attachment means (7), in particular by springes.

5. The system according to any of the preceding claims wherein the means for capturing sound is a microphone.

6. The system according to any of the preceding claims wherein the acoustic isolation box is capable of reducing external noise by at least 5 dB inside the box.

7. The system according to claim 5 or 6 wherein the microphone is connected to a computer.

8. The system according to any of the proceeding claims wherein a rod is used for the attachment of the sound generator to the guiding device, in particular via attachment means.

9. A method for evaluating skin texture using a system (10) comprising an acoustic isolation box, said isolation box (4) being equipped with at least one opening (5) for insertion of the skin to be measured, a guiding device (2) having a sound generator (6) attached thereto, the sound generator being placed within said isolation box and being adapted to generate sound when dragged across or slidably contacted with the skin, and means (8) for capturing sound, said method comprising:
a) placing a portion of skin into contact with the sound generator inside the acoustic isolation box;
b) moving the guiding device such that the sound generator slidably contacts the skin to generate sound;
c) capturing the sound; and
d) comparing the captured sound to a predetermined standard.

10. The method according to claim 9 wherein the means for capturing sound is a microphone.

11. The method according to claim 9 or 10, wherein the guiding device is moved using a motor (20).

12. The method according to anyone of claims 9 to 11, wherein the captured sound is compared to the predetermined standard using a computer.

13. The method according to anyone of claims 9 to 12 wherein the captured sound is compared to the predetermined standard using volume of sound in decibels.

14. The method according to anyone of claims 9 to 13 wherein the captured sound is compared to the predetermined standard using computer generated algorithms.

## Patentansprüche

1. Akustisches System (10) zur Bestimmung von Hauttextur, umfassend eine akustische Isolierbox (4), eine Führungsvorrichtung (2) mit einem daran befestigten Schallerzeuger (6), wobei der Schallerzeuger dafür geeignet ist, Schall zu erzeugen, wenn er über die Haut gezogen wird oder gleitbar mit der Haut in Kontakt gebracht wird, und ein Mittel (8) für das Erfassen von Schall, und wobei die Isolierbox mit zumindest einer Öffnung (5) für das Einführen der zu messenden Haut ausgestattet ist, und wobei der Schallerzeuger innerhalb der Isolierbox platziert ist.

2. System nach Anspruch 1, weiterhin einen an der Führungsvorrichtung befestigten Motor (20) umfassend.

3. System nach Anspruch 1 oder 2, **dadurch gekennzeichnet, dass** der Schallerzeuger eine Karte ist.

4. System nach Anspruch 3, **dadurch gekennzeichnet, dass** die Karte an der Führungsvorrichtung mit Hilfe eines Befestigungsmittels (7) befestigt ist, im Besonderen durch Federn.

5. System nach einem der vorangehenden Ansprüche, **dadurch gekennzeichnet, dass** das Mittel für das Erfassen von Schall ein Mikrofon ist.

6. System nach einem der vorangehenden Ansprüche, **dadurch gekennzeichnet, dass** die akustische Isolierbox in der Lage ist, externen Lärm um zumindest 5 Dezibel innerhalb der Box zu reduzieren.

7. System nach Anspruch 5 oder 6, **dadurch gekennzeichnet, dass** das Mikrofon an einen Computer angeschlossen ist.

8. System nach einem der vorangehenden Ansprüche, **dadurch gekennzeichnet, dass** ein Stab für die Befestigung des Schallerzeugers an der Führungsvorrichtung verwendet wird, im Besonderen mit Hilfe eines Befestigungsmittels.

9. Verfahren für die Bestimmung der Hauttextur, welches ein System (10) verwendet, das eine akustische Isolierbox umfasst, wobei die Isolierbox (4) mit zumindest einer Öffnung (5) für das Einführen der zu messenden Haut ausgestattet ist, eine Führungsvorrichtung (2) mit einem daran befestigten Schallerzeuger (6), wobei der Schallerzeuger innerhalb der Isolierbox platziert ist und dafür geeignet ist, Schall zu erzeugen, wenn er über die Haut gezogen wird oder gleitbar mit der Haut in Kontakt gebracht wird, und Mittel (8) für das Erfassen von Schall, wobei das Verfahren umfasst:
a) das Inkontaktbringen eines Abschnitts Haut mit dem Schallerzeuger im Inneren der akustischen Isolierbox;
b) Bewegen der Führungsvorrichtung dergestalt, dass der Schallerzeuger die Haut gleitbar kontaktiert, um Schall zu erzeugen;
c) Erfassen des Schalls; und
d) Vergleichen des erfassten Schalls mit einem vorgegebenen Standard.

10. Verfahren nach Anspruch 9, **dadurch gekennzeichnet, dass** das Mittel für das Erfassen von Schall ein Mikrofon ist.

11. Verfahren nach Anspruch 9 oder 10, **dadurch gekennzeichnet, dass** die Führungsvorrichtung unter Nutzung eines Motors (20) bewegt wird.

12. Verfahren nach einem der Ansprüche 9 bis 11, **dadurch gekennzeichnet, dass** der erfasste Schall mit dem vorgegebenen Standard unter Verwendung eines Computers verglichen wird.

13. Verfahren nach einem der Ansprüche 9 bis 12, **dadurch gekennzeichnet, dass** der erfasste Schall mit dem vorgegebenen Standard unter Nutzung der Lautstärke in Dezibel verglichen wird.

14. Verfahren nach einem der Ansprüche 9 bis 13, **dadurch gekennzeichnet, dass** der erfasste Schall mit dem vorgegebenen Standard unter Verwendung computererzeugter Algorithmen verglichen wird.

## Revendications

1. Système acoustique (10) pour évaluer une texture de peau, comprenant une boîte d'isolation acoustique (4), un dispositif de guidage (2) ayant un générateur de son (6) relié à celui-ci, le générateur de son étant adapté pour produire un son lorsqu'il est traîné à travers la peau ou en contact de manière coulissante avec la peau, et des moyens (8) de capture de son, et dans lequel la boîte d'isolation est munie d'au moins une ouverture (5) destinée à l'insertion de la peau à mesurer, et dans lequel le générateur de son est placé à l'intérieur de la boîte d'isolation.

2. Système selon la revendication 1, comprenant de plus un moteur (20) relié au dispositif de guidage.

3. Système selon la revendication 1 ou 2, dans lequel le générateur de son est une carte.

4. Système selon la revendication 3, dans lequel la carte est reliée au dispositif de guidage par des moyens de fixation (7), en particulier par des ressorts.

5. Système selon l'une quelconque des revendications précédentes, dans lequel les moyens de capture de son sont constitués d'un microphone.

6. Système selon l'une quelconque des revendications précédentes, dans lequel la boîte d'isolation acoustique est capable de réduire un bruit externe d'au moins 5 dB à l'intérieur de la boîte.

7. Système selon la revendication 5 ou 6, dans lequel le microphone est relié à un ordinateur.

8. Système selon l'une quelconque des revendications précédentes, dans lequel une tige est utilisée pour relier le générateur de son au dispositif de guidage, en particulier via des moyens de liaison.

9. Procédé pour évaluer une texture de peau en utilisant un système (10) comprenant une boîte d'isolation acoustique, ladite boîte d'isolation (10) étant munie d'au moins une ouverture (5) destinée à l'insertion de la peau à mesurer, un dispositif de guidage (2) ayant un générateur de son (6) relié à celui-ci, le générateur de son étant placé à l'intérieur de ladite boîte d'isolation et étant adapté pour produire un son lorsqu'il est traîné à travers la peau ou en contact coulissant avec la peau, et des moyens (8) de capture de son, ledit procédé comprenant les étapes consistant à :
a) mettre une partie de peau en contact avec le générateur de son à l'intérieur de la boîte d'isolation acoustique ;
b) déplacer le dispositif de guidage de telle sorte que le générateur de son soit en contact de manière coulissante avec la peau pour produire un son ;
c) capturer le son ; et
d) comparer le son capturé à un standard prédéterminé.

10. Procédé selon la revendication 9, dans lequel les moyens de capture de son sont constitués d'un microphone.

11. Procédé selon la revendication 9 ou 10, dans lequel le dispositif de guidage est déplacé en utilisant un moteur (20).

12. Procédé selon l'une quelconque des revendications 9 à 11, dans lequel le son capturé est comparé au standard prédéterminé en utilisant un ordinateur.

13. Procédé selon l'une quelconque des revendications 9 à 12, dans lequel le son capturé est comparé au standard prédéterminé en utilisant un volume de son en décibels.

14. Procédé selon l'une quelconque des revendications 9 à 13, dans lequel le son capturé est comparé au standard prédéterminé en utilisant des algorithmes produits par ordinateur.
